## Europäisches Patentamt
## European Patent Office
## Office européen des brevets

(19)

(11) Veröffentlichungsnummer: **0 013 551**
**B1**

(12) **EUROPÄISCHE PATENTSCHRIFT**

(45) Veröffentlichungstag der Patentschrift:
25.11.81

(51) Int. Cl.³: **C 07 C 67/56** // C07C67/05, C07C69/16

(21) Anmeldenummer: **80100003.5**

(22) Anmeldetag: **02.01.80**

(54) **Verfahren zur Entfernung von Jod aus einem Gemisch, das bei der oxidativen Acylierung von Olefinen erhalten wurde.**

(30) Priorität: **15.01.79 DE 2901359**

(43) Veröffentlichungstag der Anmeldung:
**23.07.80 Patentblatt 80/15**

(45) Bekanntmachung des Hinweises auf die Patenterteilung:
**25.11.81 Patentblatt 81/47**

(84) Benannte Vertragsstaaten:
**BE DE FR GB IT NL**

(56) Entgegenhaltungen:
**keine**

(73) Patentinhaber: **BASF Aktiengesellschaft,
Carl-Bosch-Strasse 38, D-6700 Ludwigshafen (DE)**

(72) Erfinder: **Platz, Rolf, Dr., Hansastrasse 5,
D-6800 Mannheim 1 (DE)**
Erfinder: **Hartig, Juergen, Dr., In der Schleit 9,
D-6718 Gruenstadt (DE)**
Erfinder: **Weitz, Hans-Martin, Dr., Auf dem Koeppel 40,
D-6702 Bad Duerkheim 1 (DE)**

# 0 013 551

Verfahren zur Entfernung von Jod aus einem Gemisch,
das bei der oxidativen Acylierung von Olefinen erhalten wurde

Diese Erfindung betrifft ein Verfahren zur Entfernung von Jod aus jodhaltigen Gemischen, die bei der oxidativen Acylierung von Olefinen mit Jod enthaltenden Katalysatoren erhalten werden.

Die Entfernung von Jod aus organischen Verbindungen stellt z. B. in den Fällen ein technisches Problem dar, in denen die Verwendung jodhaltiger Katalysatoren zu schwer zu reinigenden Verunreinigungen der Reaktionsprodukte führt. So werden beispielsweise bei der oxidativen Acylierung von Olefinen, bei der man Olefine, wie Ethen oder Propen in Gegenwart von Jod enthaltenden Katalysatoren mit Carbonsäuren behandelt, Reaktionsprodukte enthalten, die Jod chemisch gebunden enthalten, wodurch die Isolierung der reinen Carbonsäureestern des Äthylen- oder Propylenglykols sehr erschwert wird. Da sich das Halogen bei der weiteren Umsetzung der Carbonsäureester, z. B. zur Herstellung von Ethylenglykol oder bei der Pyrolyse von Propylenglykolmonoacetat zu Propylenoxid überaus störend auswirkt, war nach einem Verfahren zu suchen, das es gestattet, das Jod möglichst einfach und vollständig aus der organischen Verbindung zu entfernen. Wünschenswert war es außerdem, ein Verfahren zu finden, das es gleichzeitig erlaubt, das abgetrennte Jod ohne großen Aufwand zu isolieren oder erneut in die katalytische Reaktion zurückzuführen.

In der US-PS 2 385 483 wird ein Verfahren zur Rückgewinnung und Reinigung von Jod beschrieben, bei dem man Jod enthaltende organische Verbindungen hydrolysiert und die dabei erhaltenen jodhaltigen Salze oxidiert. Aufgearbeitet wird in Gegenwart von Schwefelsäure. Diese Methode ist aufwendig und führt zu Jodverlusten. Nach der US-PS 3 394 078 werden jodhaltige Verbindungen durch Umsetzung mit einem Alkalimetall in Dimethylsulfoxid vom Jod befreit. Auch dieses Verfahren ist umständlich und z. B. nicht für die Aufarbeitung katalytischer Reaktionen geeignet, bei denen der Katalysator erneut eingesetzt werden soll. Die gleichen Schwierigkeiten ergeben sich bei dem aus der US-PS 3 405 195 bekannten Verfahren, bei dem Jod durch Behandlung mit Hydroxiden entfernt wird sowie bei dem in der US-PS 3 884 965 beschriebenen Verfahren, bei dem organisch gebundenes Jod durch Behandlung mit bestimmten Metallen in anorganische Jodsalze übergeführt wird, eine Methode, die außerdem zu Abwasserproblemen führt. Nach den Angaben der US-PS 4 087 623 wird Jod aus einem Gemisch entfernt, das bei der oxidativen Acylierung von Olefinen mit Jod enthaltenden Katalysatoren erhalten wurde. Bei diesem Verfahren wird ein Teil des Jods durch Destillation abgetrennt. Das im Wertprodukt enthaltene Jod wird durch Reaktion mit einem Überschuß an Alkaliacetat zu Alkalijodid umgesetzt. Das Salzgemisch wird vom Produkt durch Filtration und Destillation abgetrennt. Um den Katalysator zu recyclisieren muß das Salzgemisch in Gegenwart von Carbonsäure und mindestens der stöchiometrischen Menge an wäßriger Wasserstoffperoxid-Lösung umgesetzt werden. Das freie Jod wird dann durch Sublimation abgetrennt. Abgesehen davon, daß sich hierbei nur etwa 80% des Jods zurückgewinnen lassen, ist das Verfahren technisch umständlich, insbesondere fordert es einen hohen Destillationsaufwand.

Es wurde nun gefunden, daß man Jod aus einem Gemisch, das bei der oxidativen Acylierung von Olefinen mit Jod enthaltenden Katalysatoren erhalten wurde, besonders vorteilhaft entfernen kann, wenn man das Jod enthaltende Gemisch bei 50 bis 200° C mit einem Oxidationsmittel behandelt und das Reaktionsgemisch gleichzeitig oder anschließend mit einem Adsorptionsmittel oder mit einem feinverteilten Metall in Berührung bringt.

Das neue Verfahren bewirkt eine einfache und praktisch quantitative Entfernung des Jods und außerdem eine einfache Rückgewinnung des Jods aus den genannten Gemischen, die Jod in chemischer Bindung enthalten.

Die Jod enthaltenden Gemische der genannten Art werden z. B. bei der Umsetzung von Olefinen, wie Ethen oder Propen mit Carbonsäuren, wie Essigsäure in Gegenwart von Jod enthaltenden Katalysatoren bei Temperaturen von 140 bis 210° C und Drücken über 1 bar, z. B. 10 bis 50 bar, erhalten. Als Katalysatoren werden hierbei die bekannten Jod enthaltenden Katalysatoren, die z. B. aus Übergangsmetallen, wie Tellur, Chrom, Nickel, Molbybdän oder Vanadin, deren Oxiden oder Halogeniden und elementarem Jod bestehen, und in denen das Molverhältnis Metall zu Jod, z. B. zwischen den Grenzen 1 zu 1 bis 1 zu 6 liegt, verwendet.

Die bei der genannten oxidativen Acylierung von Ethen und Propen mit Essigsäure (Acetoxilierung) z. B. unter Verwendung des Katalysatorsystems $TeO_2/J_2$ erhältlichen Reaktionsgemische enthalten z. B. nicht umgesetztes Olefin, Sauerstoff, Kohlendioxid, leichtsiedenden Verbindungen wie Aceton, i-Propylacetat, Acrolein, Allylacetat und Allylalkohol, Di- und Monoacetoxyhydroxyalkan (im Fall von Propen als Olefin Di- und Mono-acetoxypropan), Dihydroxyalkan, Di-, Tri- und Tetrahydroxy- bzw. Acetoxyäther der Glykole und Jodacetoxypropan.

Da sich das in diesen Reaktionsgemischen enthaltende freie Jod sowie ein Teil des gebundenen Jods durch Destillation mit den Leichtsiedern oder den Hochsiedern abtrennen läßt, ist es vorteilhaft, wenn man die Reaktionsgemische vor der erfindungsgemäßen Jodentfernung fraktioniert destilliert. Dabei werden die Jod enthaltenden im wesentlichen aus Essigsäure und Wasser bestehenden Leichtsieder, die z. B. bei Temperaturen bis zu 130° C (unter Normaldruck) übergehen und die im Rückstand verbleibenden Hochsieder, gegebenenfalls nach weiterer Aufarbeitung, in die

2

Acetoxylierung zurückgeführt. Das durch diese Destillation vorgereinigte Reaktionsgemisch, das bei der Destillation als Hauptfraktion im Temperaturbereich von 120 bis 195° C (bei Normaldruck) oder von 110 bis 125° C bei 125 mbar anfällt, enthält etwa 0,5 bis 6 Gew.-% Jod in gebundener Form.

Das erfindungsgemäße Verfahren wird z. B. durchgeführt indem man die Jod enthaltenden Reaktionsgemische der genannten Art bei 50 bis 200° C, vorzugsweise 100 bis 150° C, mit einem Oxidationsmittel, wie Sauerstoff (z. B. in Form von Luft) oder Wasserstoffperoxid behandelt und das Reaktionsgemisch gleichzeitig oder anschließend mit einem Adsorptionsmittel oder einem feinverteilten Metall in Berührung bringt. Vorteilhaft ist es, die oxidative Behandlung in Gegenwart des Adsorbens vorzunehmen. Als Adsorptionsmittel kommen z. B. Aktivkohle, Zeolithe oder Molekularsiebe und als feinverteiltes Metall, z. B. Kupfer, gegebenenfalls auf Trägermaterial, wie Magnesiumsilikat in Betracht. Aktivkohle ist bevorzugt. Das flüssige Reaktionsgemisch wird zweckmäßig von oben nach unten über eine Schüttung des Adsorptionsmittels geleitet, wobei man auf 1 l Adsorbens pro Stunde etwa 0,1 bis 10, vorzugsweise 0,5 bis 2 l, des Reaktionsgemisches einwirken läßt.

Die Jod enthaltenden Ausgangsstoffe werden zweckmäßig in Gegenwart von Essigsäure, die z. B. 20 bis 50 Gew.-% des Gemisches ausmachen kann, eingesetzt. Die Gemische können auch Wasser, z. B. bis zu 30 Gewichtsprozent des Gemisches enthalten.

Das Oxidationsmittel wird zweckmäßig in stöchiometrischen Mengen eingesetzt. Gegebenenfalls kann auch ein Über- oder Unterschuß angewendet werden. Der Einsatz von Wasserstoffperoxid ist bevorzugt. Dabei kann sich jedoch auch eine gleichzeitige Anwesenheit von Luft oder Sauerstoff günstig auswirken. Beispielsweise kann die Anwesenheit von 1 bis 160 l Sauerstoff/l Adsorbens und Stunde einen günstigen Einfluß auf die anzuwendenden $H_2O_2$-Mengen haben, da das Adsorbens die $H_2O_2$-Zersetzung katalysiert, wodurch sich der Verbrauch an Oxidationsmittel erhöht. Andererseits reagiert auch der Sauerstoff selbst mit der jodorganischen Verbindung unter Freisetzung von elementarem Jod.

Das durch das Adsorbens aufgenommene Jod kann durch Desorption leicht zurückgewonnen und gegebenenfalls erneut in die katalytische Reaktion zurückgeführt werden. Beispielsweise erhitzt man die mit Jod beladene Aktivkohle, zweckmäßig in Gegenwart einer Carbonsäure, wie Essigsäure und oder eines inerten Trägergases, wie Stickstoff auf 150 bis 300° C. Man erhält so eine Lösung von Jod in der Carbonsäure, die unmittelbar in die katalytische Reaktion zurückgeführt werden kann. Man kann das durch das feinverteilte Metall gebundene Jod aber auch durch eine einfache chemische Reaktion zurückgewinnen. So läßt sich z. B. das Jod von dem feinverteilten Kupfer durch Behandlung mit Wasserstoff bei Temperaturen von 300 bis 480° C in Form von Jodwasserstoff gewinnen.

Nach dem neuen Verfahren lassen sich die genannten Gemische auf einfache und besonders wirkungsvolle Weise von Jod befreien, wobei sich das abgetrennte Jod leicht zurückgewinnen läßt. Dieses vorteilhafte Ergebnis ist überraschend, weil nicht angenommen werden konnte, daß die Jod enthaltenden Carbonsäureester des Ethylen- und Propylenglykols bei den Verfahrensbedingungen gegenüber Wasserstoffperoxid beständig sind und sich eine so weitgehende Freisetzung des Jods erreichen läßt.


## Beispiel 1

500 g Essigsäure, 3,35 g $TeO_2$ und 25,25 g Jod gibt man in einen mit einem Begasungsrührer versehenen Titanautoklav. Bei 1500 U/min werden 0,4 l/min Sauerstoff und 4,0 l/min Propen eingeleitet. Bei einem Betriebsdruck von ca. 6 bar wird der Autoklav mittels Ölheizung innerhalb von weniger als 5 Minuten auf 180° C aufgeheizt. Bei einem Druck von 16 bar wird die Reaktion 2 Stunden fortgesetzt. Danach wird der Autoklaveninhalt rasch abgekühlt und entspannt. Der Autoklaveninhalt (572 g; 0,3% Aceton, 0,6% i-Propylacetat, 0,3% Allylacetat, 0,05% Allylalkohol, 23,1% Propandioldiacetat, 6,0% Propandiol-1-monoacetat, 2,4% Propandiol-2-monoacetat; 2,9% Wasser, 3,0% Hochsieder [Kp. 160°/l mbar]) wird im Vakuum bei 82 bis 108° C und 133 mbar in einer Kurzwegdestillation von den Hochsiedern befreit. Das erhaltene Destillat wird dann fraktioniert. Bei einem Einsatz von 441,3 g werden folgende Fraktionen mit einer 50-cm-Vigreux-Kolonne erhalten:

3

| Fraktion | Sumpf (°C) | Übergang (°C) | Druck (mbar) | Rücklauf (1') | Austrag (g) | Jod (%) |
|---|---|---|---|---|---|---|
| 1 | 60— 77 | 50— 55 | 133—124 | 2 : 1 | 246,8 | 2,2 |
| 2 | 77—103 | 55— 60 | 126 | 2 : 1 | | |
| 3 | 112—120 | 60—108 | 126 | 2 : 1 | 16,4 | 3,3 |
| 4 | 120—122 | 108—116 | 124 | 1 : 1 | 137,2 | 4,0 |
| Rückstand | | | | | 19,4 | 4,6 |

Kühlfalle und Probenmenge 19,6 g

25,0 g der Fraktion 4 (66,4% Propandioldiacetat, 22,7% Propandiolmonoacetate, 0,11% Wasser; 1,8% Essigsäure; 4,0% Gesamtjod) werden zusammen mit 25,0 g Essigsäure und 1,5 g $H_2O_2$ (20%ig) bei einer Temperatur von 100°C innerhalb von 2 Stunden von oben über eine Schüttung von 10,0 g Aktivkohle, die im Handel unter dem eingetragenen Warenzeichen HYDRAFFIN erhältlich ist (Typ BS 12, ca. 30 ml), geleitet. Im ablaufenden Produkt bzw. auf der Kohle wird der Jodgehalt bestimmt. Es wird festgestellt, daß nur noch 0,026% Jod im Austrag vorhanden sind. Dieses bedeutet, daß mehr als 97% des Jods entfernt wurden. Die Jod enthaltende Aktivkohle wird im schwachen $N_2$-Strom unter Zusatz von 5 g/h Essigsäure auf 250°C erhitzt. Die austretenden Joddämpfe werden durch Essigsäure geleitet. Nach 2 Stunden wird festgestellt, daß 97,5% des adsorbierten Jods in der Essigsäure vorliegen. Das Jod/Essigsäure-Gemisch wird recyclisiert.

### Beispiel 2

Man verfährt wie in Beispiel 1 beschrieben, wobei man als Fraktion 4 einen Acetatschnitt erhält, der 5,0% Jod enthält. 25,0 g dieses Gemisches werden zusammen mit 75 g Essigsäure, 20 g Wasser und 1,5 g $H_2O_2$ (20%ig) innerhalb von 2 Stunden über 10 g Aktivkohle (Hydraffin BS 12) in Anwesenheit von 3 l/h Sauerstoff geleitet. Das ablaufende Produkt enthält noch 0,079% Jod (92,4%ige Entfernung).

### Beispiel 3

97 g des aus Beispiel 2 erhaltenen Acetatgemisches mit einem Jodgehalt von 0,079% werden zusammen mit 1,5 g $H_2O_2$ (20%ig) bei 200°C und innerhalb 2 Stunden über 10 g eines Cu-Trägerkatalysators (Typ R3-11; 5-mm-∅-Stränge, bei 200°C und 2 h reduziert; Zusammensetzung in oxidischer Form: 36,5% Cu, 14,4- Mg, 28,5% $SiO_2$) geleitet. Im Austrag sind noch 0,004% Jod enthalten. Dieses bedeutet eine Entjodierung von 99,5%, bezogen auf das Destillat des Reaktoraustrages. Durch Überleiten von Wasserstoff kann das Jod in Form von Jodwasserstoff vom Katalysator entfernt und in die Synthese zurückgeführt werden.

**Patentansprüche**

1. Verfahren zur Entfernung von Jod aus einem Gemisch, das bei der oxidativen Acylierung von Olefinen mit Jod enthaltenden Katalysatoren erhalten wurde, dadurch gekennzeichnet, daß man das Jod enthaltende Gemisch bei 50 bis 200°C mit einem Oxidationsmittel behandelt und das Reaktionsgemisch gleichzeitig oder anschließend mit einem Adsorptionsmittel oder mit einem feinverteilten Metall in Berührung bringt.

2. Verfahren nach Anspruch 1, dadurch gekennzeichnet, daß man als Oxidationsmittel Sauerstoff oder Wasserstoffperoxid und als Adsorptionsmittel Aktivkohle verwendet.

3. Verfahren nach Anspruch 1, dadurch gekennzeichnet, daß man das durch das Adsorbens adsorbierte Jod desorbiert und für eine durch Jod katalysierte oxidative Acylierung wiederverwendet.

4. Verfahren nach Anspruch 1, dadurch gekennzeichnet, daß man als Oxidationsmittel Wasserstoffperoxid zusammen mit Sauerstoff verwendet.

## Claims

1. A process for removing iodine from a mixture which has been obtained in the oxidative acylation of olefins using iodine-containing catalysts, characterized in that the iodinecontaining mixture is treated with an oxidizing agent at from 50 to 200°C and, simultaneously or subsequently, the reaction mixture is brought into contact with an adsorbent or a finely divided metal.

2. A process as claimed in claim 1, characterized in that oxygen or hydrogen peroxide is used as the oxidizing agent and active charcoal is used as the adsorbent.

3. A process as claimed in claim 1, characterized in that the iodine adsorbed by the adsorbent is desorbed and re-used for an iodine-catalyzed oxidative acylation.

4. A process as claimed in claim 1, characterized in that hydrogen peroxide together with oxygen is used as the oxidizing agent.

## Revendications

1. Procédé pour éliminer l'iode contenu dans un mélange obtenu à l'acylation oxydante des oléfines sur des catalyseurs contenant de l'iode, caractérisé en ce que l'on traite les mélanges contenant de l'iode à une température de 50 à 200°C par un agent oxydant et on met le mélange de réaction en contact simultanément ou par la suite avec un agent adsorbant ou avec un métal à l'état de fine division.

2. Procédé selon la revendication 1, caractérisé en ce que l'agent oxydant utilisé est l'oxygène ou le peroxyde d'hydrogène et l'agent adsorbant le charbon actif.

3. Procédé selon la revendication 1, caractérisé en ce que l'iode adsorbé par l'adsorbant est désorbé et réutilisé dans une acylation oxydante catalysée par l'iode.

4. Procédé selon la revendication 1, caractérisé en ce que l'on utilise en tant qu'agents oxydants le peroxyde d'hydrogène avec l'oxygène.